# EUROPEAN PATENT APPLICATION

(11) **EP 4 219 409 A1**
(43) Date of publication of application: **02.08.2023**
(21) Application number: 22784821.5
(22) Date of filing: 24.03.2022
(51) Int. Cl.: C02F 1/467, C02F 1/461, C02F 1/00

(54) **STERILIZED WATER GENERATOR, WATER PURIFIER AND METHOD FOR CONTROLLING SAME**

(30) Priority: 09.04.2021 KR 20210046670
(71) Applicant: Samsung Electronics Co., Ltd., Suwon-si, Gyeonggi-do 16677 (KR)
(72) Inventor: PARK, Jungha, Suwon-si Gyeonggi-do 16677 (KR); LEE, Junggeun, Suwon-si Gyeonggi-do 16677 (KR); KANG, Wanku, Suwon-si Gyeonggi-do 16677 (KR); LEE, Jongho, Suwon-si Gyeonggi-do 16677 (KR)
(74) Representative: Gulde & Partner
(86) International application number: PCT/KR2022/004122
(87) International publication number: WO 2022/215911

(57) **Abstract**

A sterilized water generator to control some of a plurality of electrolysis modules not to perform electrolysis on water brought into the sterilized water generator. The sterilized water generator includes a water inlet pipe through which water flows in; a water outlet pipe through which sterilized water flows out; a plurality of electrolysis modules arranged in parallel between the water inlet pipe and the water outlet pipe and configured to turn the water brought in through the water inlet pipe to the sterilized water; and a controller configured to control a forward voltage not to be applied to a first electrolysis module of the plurality of electrolysis modules, control the forward voltage to be applied to a second electrolysis module of the plurality of electrolysis modules, and change electrolysis modules corresponding to the first and second electrolysis modules of the plurality of electrolysis modules based on a lapse of time.

## Description

### [Technical Field]

The disclosure relates to a sterilized water generator capable of controlling a plurality of electrolysis modules not to electrolyze water rotationally, and a water purifier including the sterilized water generator.

### [Background Art]

A sterilized water generator is a device for generating sterilized water by electrolyzing water brought in and providing the sterilized water for a user or a device that uses water such as a water purifier, a dish washer, a washing machine, etc.

The sterilized water generator may include an electrolysis module for performing electrolysis to generate sterilized water, in which case the water brought into the sterilized water generator may be electrolyzed by the electrolysis module and turned to sterilized water including a sterilizing material.

In this case, when the water flows fast while passing the sterilized water generator, a reaction time of the water in the electrolysis module may be shortened, which may degrade the electrolysis performance and lower the concentration of the sterilizing material. On the contrary, when the water flows slowly while passing the sterilized water generator, usability for the user becomes bad.

To maintain the flow velocity of the water passing the sterilized water generator but increase the flow volume, widening a gap between electrodes is under consideration, but when the gap between the electrodes is widened, the efficiency of electrolysis is reduced.

### [Disclosure]

### [Technical Problem]

The disclosure provides a sterilized water generator that is able to control some of a plurality of electrolysis modules not to perform electrolysis on water brought into the sterilized water generator, thereby stably securing flow volume of sterilized water and increasing a lifespan of each electrolysis module.

### [Technical Solution]

According to an embodiment of the disclosure, a sterilized water generator includes a water inlet pipe through which water flows to the sterilized water generator; a water outlet pipe through which sterilized water flows from the water generator; a plurality of electrolysis modules arranged in parallel to each other, along a flow path, between the water inlet pipe and the water outlet pipe, the plurality of electrolysis modules configured to electrolyze the water flowing through the water inlet pipe to produce the sterilized water; and a controller configured to control a first forward voltage so that the first forward voltage is not applied to a first electrolysis module of the plurality of electrolysis modules, control a second forward voltage so that the second forward voltage is applied to a second electrolysis module of the plurality of electrolysis modules, and change the first forward voltage of the first electrolysis module and change the second forward voltage of the second electrolysis module based on a predetermined lapse of time.

The controller may control a reverse voltage so that the reverse voltage is applied to an electrode of the first electrolysis module.

The controller may control a voltage so that the voltage is not applied to an electrode of the first electrolysis module.

The controller may control a forward voltage so that the forward voltage is not applied to the plurality of electrolysis modules rotationally.

The sterilized water generator may further include a sensor module for measuring a condition of the water flowing through the water inlet pipe, and the controller may determine a number of first electrolysis modules based on the condition of water flowing through the water inlet pipe, and control the first forward voltage so that the first forward voltage is not applied to the determined number of first electrolysis modules.

The sensor module may measure an amount of total dissolved solids (TDSs) in the water flowing through the water inlet pipe, and the controller may determine a number of the first electrolysis modules based on the amount of TDSs in the water flowing through the inlet pipe.

The sensor module may measure water temperature of the water flowing through the water inlet pipe, and the controller may determine a number of the first electrolysis modules based on the water temperature of the water flowing through the water inlet pipe.

The sensor module may measure the magnitude of a current flowing in the water passing the plurality of electrolysis modules, and the controller may determine a number of the first electrolysis modules based on the magnitude of the current.

The sterilized water generator may further include a communication module for receiving outside environment information from a server, and the controller may determine a number of the first electrolysis modules based on the outside environment information, and control the first forward voltage so that the first forward voltage is not applied to the number of first electrolysis modules.

The communication module may receive, from the server, weather information of a region in which the sterilized water generator is located, and the controller may determine a number of the first electrolysis modules based on the weather information.

The communication module may receive, from the server, water quality information of a region in which the sterilized water generator is located, and the controller may determine a number of the first electrolysis modules based on the water quality information.

The sterilized water generator may further include a flow volume sensor for detecting flow volume of the water brought in, and when the flow volume of the sterilized water flowing out after the water is electrolyzed is equal to or greater than a reference flow volume, the controller may control a second forward voltage not to be applied to the first electrolysis module of the plurality of electrolysis modules and control the forward voltage to be applied to the second electrolysis module of the plurality of electrolysis modules.

The sterilized water generator may further include a display, and the controller may control the display to display an indication of an electrolysis module required to be replaced among the plurality of electrolysis modules.

The sterilized water generator may further include a notifier, and the controller may control the notifier to output an alarm indicating an electrolysis module causing a malfunction or fault among the plurality of electrolysis modules.

According to an embodiment of the disclosure, a water purifier includes a first connection flow path through which water flows to the water purifier; a second connection flow path through which water flows from the water purifier; a filter arranged along the first connection flow path to filter the water; and a sterilized water generator for generating sterilized water, wherein the sterilized water generator includes a water inlet pipe connected to the first connection flow path; a water outlet pipe connected to the second connection flow path; a plurality of electrolysis modules arranged parallel to each other, between the water inlet pipe and the water outlet pipe and the plurality of electrolysis modules configured to electrolyze the water flowing through the water inlet pipe to generate the sterilized water; and a controller configured to control a first forward voltage so that the first forward voltage is not applied to a first electrolysis module of the plurality of electrolysis modules, control a second forward voltage so that the second forward voltage is applied to a second electrolysis module of the plurality of electrolysis modules, and change the first forward voltage of the first electrolysis module and change the second forward voltage of the second electrolysis module based on a predetermined lapse of time.

### [Advantageous Effects]

According to an embodiment of the disclosure, a sterilized water generator may determine the number of electrolysis modules that do not perform electrolysis based on a condition of tap water or outside environment information and control a plurality of electrolysis modules not to electrolyze water brought in rotationally, thereby stably securing flow volume of sterilized water, increasing a lifespan of each electrolysis module, and maintaining a certain level of electrolysis performance.

### [Description of Drawings]

FIG. 1 illustrates a sterilized water generator, according to an embodiment of the disclosure;
FIG. 2 illustrates a water purifier including a sterilized water generator, according to an embodiment of the disclosure;
FIG. 3 illustrates electrolysis performed by an electrolysis module and application of a reverse voltage to the electrolysis module, according to an embodiment of the disclosure;
FIG. 4 is a control block diagram of a sterilized water generator, according to an embodiment of the disclosure;
FIG. 5 is a table representing an example of applying a reverse voltage to one of a plurality of electrolysis modules rotationally, according to an embodiment of the disclosure;
FIG. 6 is a table representing an example of not applying a voltage to an electrode of one of a plurality of electrolysis modules rotationally, according to an embodiment of the disclosure;
FIG. 7 illustrates positions of respective sensors in a sterilized water generator, according to an embodiment of the disclosure;
FIG. 8 is a table representing an example of applying a reverse voltage to two of a plurality of electrolysis modules rotationally, according to an embodiment of the disclosure;
FIG. 9 is a table representing an example of not applying a voltage to electrodes of two of a plurality of electrolysis modules rotationally, according to an embodiment of the disclosure;
FIG. 10 illustrates a display displaying an indication of one of a plurality of electrolysis modules required to be replaced, according to an embodiment of the disclosure;
FIG. 11 is a flowchart illustrating a procedure for determining the number of first electrolysis modules based on a total dissolved solid (TDS) value in tap water, according to an embodiment of the disclosure;
FIG. 12 is a flowchart illustrating a procedure for determining the number of first electrolysis modules based on water temperature of tap water, according to an embodiment of the disclosure;
FIG. 13 is a flowchart illustrating a procedure for determining the number of first electrolysis modules based on a current flowing in each electrolysis module, according to an embodiment of the disclosure; and
FIG. 14 is a flowchart illustrating a procedure for determining the number of first electrolysis modules based on outside environment information received from a server, according to an embodiment of the disclosure.

### [Modes of the Invention]

Embodiments and features as described and illustrated in the disclosure are merely examples, and there may be various modifications replacing the embodiments and drawings at the time of filing this application.

It will be further understood that the term "connect" or its derivatives refer both to direct and indirect connection, and the indirect connection includes a connection over a wireless communication network.

The terminology used herein is for the purpose of describing particular embodiments only and is not intended to limit the disclosure. It is to be understood that the singular forms "a," "an," and "the" include plural references unless the context clearly dictates otherwise. It will be further understood that the terms "comprise" and/or "comprising," when used in this specification, specify the presence of stated features, integers, steps, operations, elements, and/or components, but do not preclude the presence or addition of one or more other features, integers, steps, operations, elements, components, and/or groups thereof.

The terms including ordinal numbers like "first" and "second" may be used to explain various components, but the components are not limited by the terms. The terms are only for the purpose of distinguishing a component from another. Thus, a first element, component, region, layer or room discussed below could be termed a second element, component, region, layer or section without departing from the teachings of the disclosure.

Furthermore, the terms, such as "~ part", "~ block", "~ member", "~ module", etc., may refer to a unit of handling at least one function or operation. For example, the terms may refer to at least one process handled by hardware such as field-programmable gate array (FPGA)/application specific integrated circuit (ASIC), etc., software stored in a memory, or at least one processor.

Reference numerals used for method steps are just used to identify the respective steps, but not to limit an order of the steps. Thus, unless the context clearly dictates otherwise, the written order may also be practiced otherwise.

Reference will now be made in detail to embodiments of the disclosure, which are illustrated in the accompanying drawings.

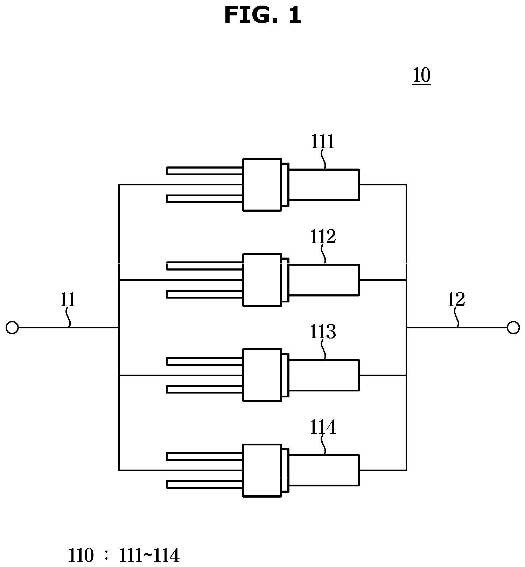

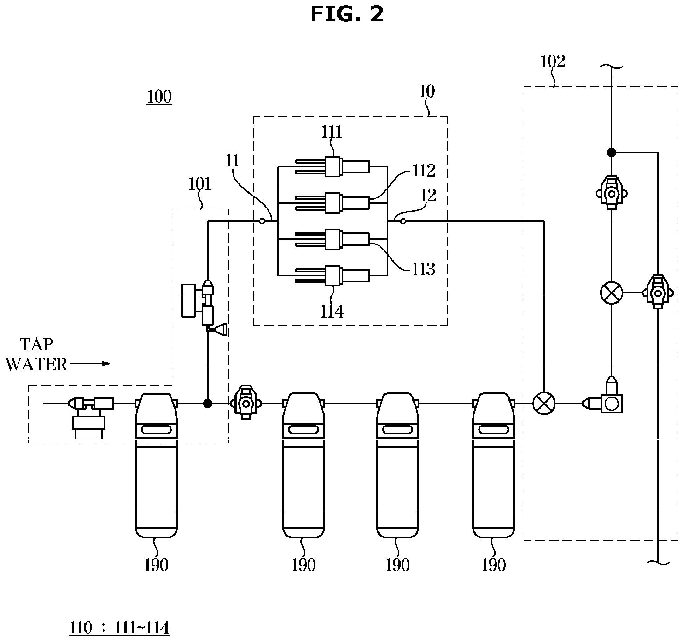

FIG. 1 illustrates a sterilized water generator, according to an embodiment of the disclosure, and FIG. 2 illustrates a water purifier, according to an embodiment of the disclosure.

Referring to FIG. 1, a sterilized water generator 10 in an embodiment of the disclosure may include a water inlet pipe 11 through which water is brought in, a water outlet pipe 12 through which sterilized water flows out, and electrolysis modules 110 for generating sterilized water by electrolyzing the water brought in.

The electrolysis modules 110 may include a plurality of plate-shaped electrodes for generating sterilizing materials by electrolyzing water.

Specifically, the electrolysis module 110 may include a plurality of plate-shaped electrodes formed of titanium and having surfaces coated with active materials such as iridium, ruthenium, platinum, etc.

The electrolysis module 110 may generate sterilizing materials such as hypochlorous acid, radical, etc., by applying positive and negative electricity to the respective plate-shaped electrodes to electrolyze water passing through or staying in a gap between the plate-shaped electrodes.

In this case, the electrolysis module 110 may be provided in the plural, and the plurality of electrolysis modules 111, 112, 113 and 114 (collectively, 110) may be arranged in parallel between the water inlet pipe 11 and the water outlet pipe 112. In other words, the plurality of electrolysis modules 110 may be provided to bring in water in parallel between the water inlet pipe 11 and the water outlet pipe 12. For example, the electrolysis module 110 may include an electrolysis module A 111, an electrolysis module B 112, an electrolysis module C, and an electrolysis module D 114.

A traditional sterilized water generator includes a single electrolysis module. The electrolysis module is a device for generating sterilized water by electrolyzing water brought into the water purifier.

In the meantime, the electrolysis module 110 applies a current to the plurality of plate-shaped electrodes generally coated with an active material to electrolyze the water flowing between the electrodes and produces chlorine and ion materials, which have sterilizing power.

In general, as for performance of the electrolysis module, the faster the flow velocity of water passing the electrolysis module, the slower the response time, causing electrolysis performance to be degraded, i.e., causing reduction in concentration of the sterilizing material. On the contrary, when the flow velocity of water passing the electrolysis module is reduced for securing the response time, a problem arises that the sterilized water is discharged slowly when the user wants to use the sterilized water.

Furthermore, even a method of widening a gap between electrodes of the electrolysis module to increase flow volume while maintaining the flow velocity of the water has a limit because the electrolysis efficiency decreases when the gap between the electrodes is widened.

To maintain electrolysis efficiency while making the flow velocity fast, a method of increasing the voltage to be applied to the electrolysis module may also have a limit on the allowable current and electric energy and shorten a lifespan of the electrolysis module because ion materials of the tap water adhere to the electrode surfaces to accelerate generation of scales including such materials as calcium (Ca) and magnesium (Mg).

Hence, in the embodiment of the disclosure, the sterilized water generator 10 may have the plurality of electrolysis modules 111 to 114, collectively, 110, arranged in parallel to secure flow volume as well as maximize electrolysis efficiency.

The plurality of electrolysis modules 110 may allow plenty of water to pass at a time even without increasing the flow velocity, thereby increasing the flow volume, as compared to when there is a single electrolysis module. Accordingly, in the embodiment of the disclosure, it is possible to secure stable flow volume when the user wants to use the sterilized water.

In this case, there is no limitation on the number of electrolysis modules 110 included in the sterilized water generator 10, and in the following description, assume that there are, for example, four electrolysis modules 111 to 114, for convenience of explanation.

The sterilized water generator 10 may be provided as a separate module, which may be inserted to a device that uses water such as a water purifier, a dish washer, a washing machine, etc., and may serve to provide sterilized water in the device.

For example, the sterilized water generator 10 may be used by being inserted to the air purifier 100, as shown in FIG. 2.

The sterilized water generator 10 may be connected to a first connection flow path 101 of the water purifier 100 through the water inlet pipe 11 and to a second connection flow path 102 through the water outlet pipe 12.

The sterilized water generator 10 may receive water, which has passed the first connection flow path 101, through the water inlet pipe 11, generate sterilized water by electrolyzing the water, and discharge the sterilized water through the water outlet pipe 12. The sterilized water discharged through the water outlet pipe 12 may be provided for the user by being released out of the air purifier 100 through the second connection flow path 102.

Tap water may be brought into the first connection flow path 101 from the outside of the water purifier 100. A tap water valve for controlling inflow of the tap water and a decompression valve for reducing pressure of the tap water may be arranged in the first connection flow path 101.

The second connection flow path 102 may discharge the water from inside of the water purifier 100 to the outside.

A water outlet nozzle and a drain port through which to discharge liquids in the water purifier 100 to the outside may be arranged at an end of the second connection flow path 102.

A water outlet valve for controlling a flow of purified water may be arranged at a front end of the water outlet nozzle. Specifically, when the water outlet valve is opened, the sterilized water generated in the sterilized water generator 10 may be discharged to the outside through the water outlet nozzle arranged at a downstream end of the second connection flow path 102.

In an embodiment of the disclosure, upstream, downstream, and front and rear ends of the air purifier 100 are determined based on a direction in which the water brought into the air purifier 100 flows. A side near where the tap water is brought in from the outside is upstream or the front end, and a side near where water is discharged is downstream or the rear end.

A filter 190 for filtering the tap water may be arranged in the first connection flow path 101.

The water purifier 100 may provide sterilized water generated by electrolyzing the tap water in the electrolysis module 110 or sterilized water generated by electrolyzing the water already filtered by the filter 190 in the electrolysis module 110, depending on the user's choice.

The filter 190 may include a plurality of filters, which may include e.g., a pre-carbon filter that adsorbs volatile materials such as chlorine and chlorine byproducts from the tap water, a membrane filter that filters off very fine contaminants by reverse osmosis, and a post-carbon filter that affects the taste of the purified water discharged.

In this case, the plurality of filters may be connected in the sequence of the pre-carbon filter, the membrane filter, and the post-carbon filter, and the tap water brought into the filter 190 may be filtered by sequentially passing the pre-carbon filter, the membrane filter, and the post-carbon filter.

These types of filters are merely an example to be applied to embodiments of the water purifier 100, and there are no limitations on the types of filters. It is also possible that different types and different numbers of filters may be provided in different arrangement.

For example, a sediment filter for filtering out sediments contained in the tap water or a high-turbidity filter for filtering out relatively coarse-grained particles may be further arranged in the first connection flow path 101.

In an embodiment of the disclosure, the water purifier 100 may further include a cold/hot water device for providing cold water or hot water, and the cold/hot water device may include a heat exchanger. The cold/hot water device may be arranged in a downstream portion of the second connection flow path 102.

It is also possible to use the sterilized water generator 10 alone. In this case, the sterilized water generator 10 may be connected through the water inlet pipe 11 to a tap water flow path through which to supply tap water, and may provide sterilized water for the user through the water outlet pipe 12.

How the sterilized water generator 10 controls the electrolysis module 110 to improve sterilization performance, sterilized water flow volume provided, and a lifespan of the electrolysis module 110 will now be described in detail.

An electrolysis procedure in the electrolysis module 110 will be described first.

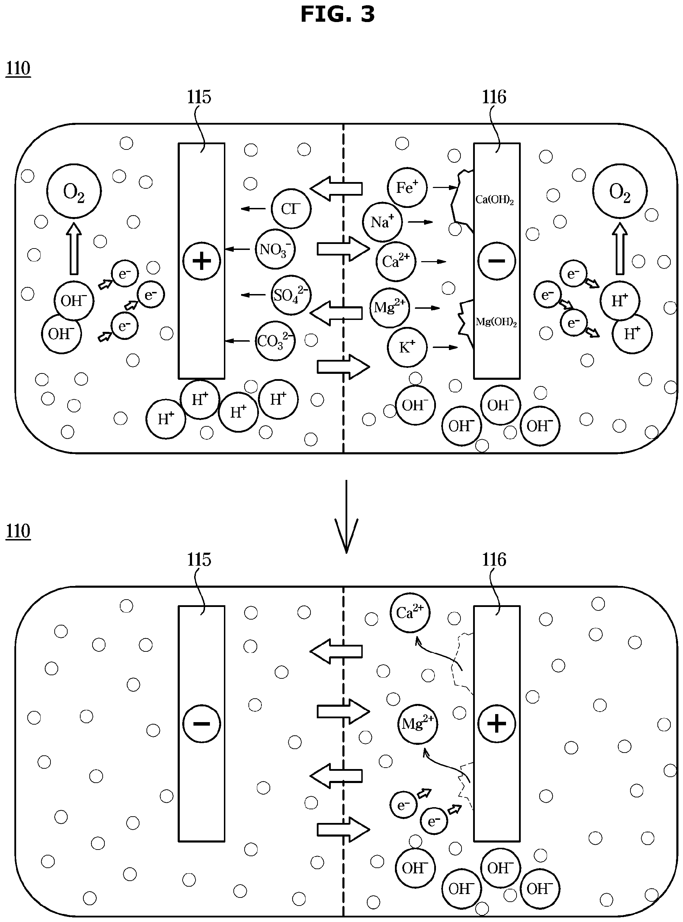

FIG. 3 illustrates electrolysis performed by the electrolysis module 110 and application of a reverse voltage to the electrolysis module 110, according to an embodiment of the disclosure.

Referring to FIG. 3, the electrolysis module 110 may include a first electrode 115 and a second electrode 116.

To electrolyze water brought in, the electrolysis module 110 may apply a positive (+) voltage to the first electrode 115 to make the first electrode 115 become a positive electrode, and apply a negative (-) voltage to the second electrode 116 to make the second electrode 116 become a negative electrode.

Positive ions (or cations) contained in the water may move to the negative electrode. For example, calcium Ca2+ ions or magnesium Mg2+ ions contained in the water may move to the second electrode 116, which is the negative electrode.

Negative ions (or anions) contained in the water may move to the positive electrode. For example, chlorine Cl- ions contained in the water may move to the first electrode 115, which is the positive electrode.

When a voltage is applied across the electrodes, hydrogen ions H+ may be produced at the positive electrode and hydroxyl ions OH- ions may be produced at the negative electrode. The hydroxyl ions OH- may be combined with e.g., hard cations to form scales on surfaces of the negative electrode. For example, the hydroxyl ions OH- may be combined with e.g., Ca2+, Mg2+, etc., to form scales such as Ca(OH)2, Mg(OH)2 or the like on surfaces of the negative electrode.

These scales formed on the surfaces of the electrode may significantly degrade ion adsorption capacity of the electrode, leading to degrading electrolysis efficiency of the electrolysis module 110.

In an embodiment of the disclosure, the electrolysis module 110 may apply a reverse voltage across the electrodes. Specifically, the electrolysis module 110 may apply a positive voltage to the first electrode 115 and apply a negative voltage to the second electrode 116 to perform electrolysis, and on the contrary, may apply the negative voltage to the first electrode 115 to make the first electrode 115 become a negative electrode and apply the positive voltage to the second electrode 116 to make the second electrode 116 become a positive electrode in order to eliminate the scales.

Electrons e- contained in the water may move to the second electrode 116 that has been turned to the positive electrode, and upon receiving the electrons e-, scales on the surfaces of the second electrode 116 may be decomposed back into hard cations and hydroxyl ions OH-.

As such, the scales that degrade efficiency of the electrolysis module 110 may be eliminated by applying a reverse voltage across the electrodes.

However, the reverse voltage needs to be applied during the time when the sterilized water is not in use, and excessive application of the reverse voltage may have a problem that might cause deterioration of the electrode. Furthermore, another problem may arise that scale fragments having fallen off at some point may be exposed to the user.

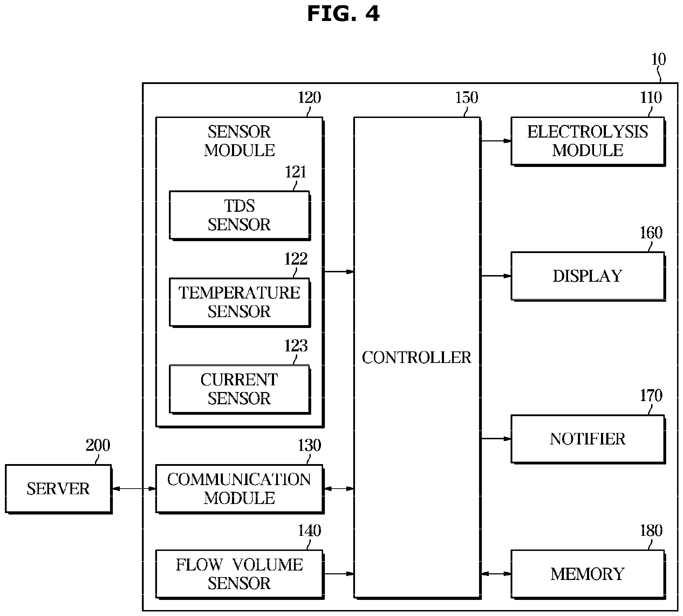

FIG. 4 is a control block diagram of the sterilized water generator 10, according to an embodiment of the disclosure.

Referring to FIG. 4, the sterilized water generator 10 may include the electrolysis module 110, a sensor module 120, a communication module 130, a flow volume sensor 140, a controller 150, a display 160, a notifier 170, and a memory 180.

The sensor module 120 may measure a condition of water brought in through the water inlet pipe 11. Specifically, the sensor module 120 may measure an amount of total dissolved solids (TDSs) in water and water temperature of the water. Furthermore, the sensor module 120 may measure the magnitude of a current flowing in the water.

The sensor module 120 may include a TDS sensor 121, a temperature sensor 122, and a current sensor 123.

The communication module 130 may receive outside environment information from a server 200. The communication module 130 may send the received outside environment information to the controller 150.

The outside environment information may include weather information in a region where the sterilized water generator 10 is located, season information in the region where the sterilized water generator 10 is located, temperature information in the region where the sterilized water generator 10 is located, and water quality information in the region where the sterilized water generator 10 is located.

The communication module 130 may receive various signals and information from the server 200 over a wireless communication network. The wireless communication network refers to a communication network over which radio signals are transmitted or received. For example, the wireless communication network may include a third generation (3G) communication network, a fourth generation (4G) communication network, a fifth generation (5G) communication network, a wireless fidelity (Wi-Fi) communication network, etc., without being limited thereto.

The flow volume sensor 140 may detect flow volume of the water brought into the sterilized water generator 10.

The display 160 may display an indication of one of the plurality of electrolysis modules 110 that needs to be replaced.

The notifier 170 may output an alarm indicating the one of the plurality of electrolysis modules 110 that has caused a malfunction or a fault.

The notifier 170 may output the alarm by displaying the alarm on the display 160 provided in the sterilized water generator 10, or output the alarm by a sound through a speaker equipped in the sterilized water generator 10.It is not, however, limited thereto, and the notifier 170 may employ any method that may indicate the electrolysis module 110 that has caused a malfunction or a fault among the plurality of electrolysis modules 110.

The controller 150 may determine at least one of the plurality of electrolysis modules 110 as a first electrolysis module and the rest of the plurality of electrolysis modules 110 as second electrolysis modules.

When the electrolysis module 110 is not determined as the first electrolysis module or is determined as the second electrolysis module, the water brought in through the water inlet pipe 11 may be electrolyzed to produce sterilized water. That is, the electrolysis module 110 may turn the water brought in through the water inlet pipe 11 to sterilized water by applying a forward voltage.

The electrolysis module 110 determined as the first electrolysis module may not electrolyze the water brought in.

The controller 150 may control the first electrolysis module of the plurality of electrolysis modules 110 not to electrolyze the water brought in, and control the second electrolysis module of the plurality of electrolysis modules 110 to electrolyze the water brought in. Specifically, the controller 150 may control the forward voltage not to be applied to the first electrolysis module of the plurality of electrolysis modules 110 and control the forward voltage to be applied to the second electrolysis module of the plurality of electrolysis modules 110.

That is, some of the plurality of electrolysis modules 110 may generate sterilized water by electrolyzing water and some of them may not electrolyze the water.

That the first electrolysis module does not electrolyze the water brought in is because a reverse voltage is applied across the electrodes of the first electrolysis module. In other words, a voltage, which is reverse to the voltage ordinarily applied to each electrode to perform electrolysis, is applied to the electrode of the electrolysis module 110 determined as the first electrolysis module to eliminate scales formed on surfaces of the electrode.

The controller 150 may control the reverse voltage to be applied to the electrode of the first electrolysis module.

In this way, a reverse voltage may be applied to electrodes of some of the plurality of electrolysis modules 110, so that the scale fragments having fallen off from the electrodes may be less exposed to the user even when the user uses the water purifier 100 during elimination of the scales formed on surfaces of the electrodes of the electrolysis module 110.

As the reverse voltage is only applied to electrodes of some of the electrolysis modules 110 while the remaining electrolysis modules 110 are generating sterilized water, the water mixed with the scale fragments after passing the electrolysis module 110 to which the reverse voltage is applied, and the sterilized water generated by the remaining electrolysis modules 110 are mixed and provided for the user. In this case, the water mixed with the scale fragments may be thinned. Accordingly, application of the reverse voltage for eliminating the scales may not need to avoid the time for which the sterilized water generator 10 is in use. Furthermore, the user may use the sterilized water generator 10 at any time no matter whether the sterilized water generator 10 is applying the reverse voltage to the electrode.

Alternatively, the first electrolysis module may not electrolyze the water brought in because no voltage is applied to the electrode of the first electrolysis module. Specifically, the electrolysis module 110 determined as the first electrolysis module may force the water brought in to pass through the water outlet pipe 12 without performing any chemical process on the water brought into the electrolysis module 110 through the water inlet pipe 11.

The controller 150 may control no voltage to be applied to the electrode of the first electrolysis module.

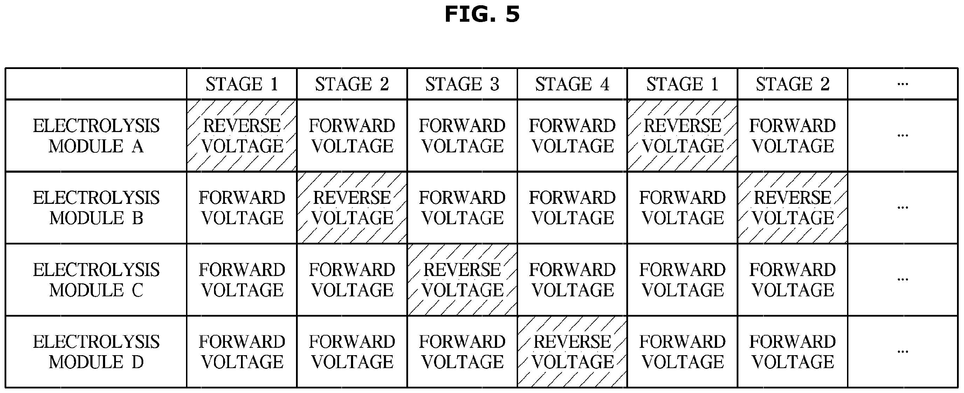

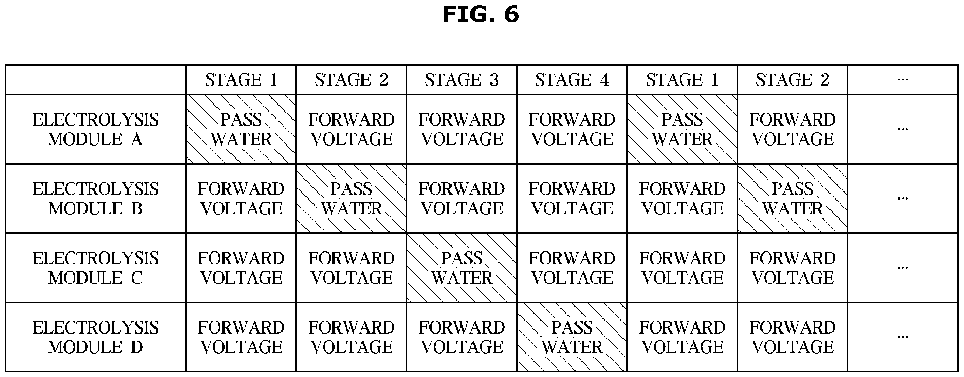

FIG. 5 is a table representing an example of applying a reverse voltage to one of the plurality of electrolysis modules 110 rotationally, according to an embodiment of the disclosure, and FIG. 6 is a table representing an example of not applying a voltage to an electrode of one of the plurality of electrolysis modules 110 rotationally, according to an embodiment of the disclosure.

Referring to FIGS. 5 and 6, the controller 150 may control the plurality of the electrolysis module 110 rotationally not to electrolyze the water brought in.

Specifically, the controller 150 may determine some of the plurality of electrolysis modules 110 as the first electrolysis module(s) and the rest of them as the second electrolysis module(s), and after the lapse of a certain time, change the electrolysis module determined as the first electrolysis module to the second electrolysis module and the electrolysis module determined as the second electrolysis module to the first electrolysis module. In this regard, the controller 150 may also maintain the electrolysis module determined as the first electrolysis module and the electrolysis module determines as the second electrolysis module as they are.

In other words, the controller 150 may rotationally change the plurality of electrolysis modules 110 to be the first and second electrolysis modules based on the lapse of time.

A certain time taken for the controller 150 to change the first and second electrolysis modules after determining the first and second electrolysis modules may be about 0.1 second, without being limited thereto.

A point in time at which the controller 150 changes the first and second electrolysis modules after determining the first and second electrolysis modules may be determined not based on the lapse of time but based on flow volume measured by the flow volume sensor 140.

In this case, from when the controller 150 determines the first and second electrolysis modules, the flow volume sensor 140 may measure flow volume of water that has passed the electrolysis module 110 and redetermine the first and second electrolysis modules when the measured flow volume exceeds a preset flow volume.

Referring to FIG. 5, the controller 150 may determine one of the plurality of electrolysis modules 110 as the first electrolysis module and the rest of them as the second electrolysis modules. In this case, the controller 150 may rotationally change one of the plurality of electrolysis modules 110 to be the first electrolysis module.

For example, when there are four electrolysis modules 110: an electrolysis module A 111, an electrolysis module B 112, an electrolysis module C 113 and an electrolysis module D 114, the reverse voltage may be applied across electrodes of one of the four electrolysis modules 110 in each stage.

In stage 1, when the reverse voltage is applied to the electrolysis module A 111, the remaining electrolysis modules 110 receive the forward voltage to generate sterilized water. Specifically, in stage 1, the electrolysis module A 111 may be the first electrolysis module, and the electrolysis modules B, C and D 112, 113 and 114 may be the second electrolysis modules.

After determining the first and second electrolysis modules in stage 1, the procedure may proceed to stage 2 after the lapse of a certain time.

In stage 2, the controller controls the reverse voltage to be applied to the electrolysis module B 112 and the forward voltage to be applied to the remaining electrolysis modules 110 to generate the sterilized water. The procedure may proceed to stage 3 after the lapse of the certain time. Specifically, in stage 2, the electrolysis module B 112 may be the first electrolysis module, and the electrolysis modules A, C and D 111, 113 and 114 may be the second electrolysis modules.

After the controller 150 determines the first and second electrolysis modules in stage 4, the procedure may proceed back to stage 1 after the lapse of the certain time. In this way, the controller 150 may control the plurality of electrolysis modules 110 to take turns to receive the reverse voltage not to perform electrolysis on the water brought in while the sterilized water generator 10 is generating sterilized water.

Meanwhile, after the reverse voltage is applied to the electrolysis module A 111, it is not always needed to apply the reverse voltage to the electrolysis module B 112 in the next stage but there may be any different sequence of applying the reverse voltage to the plurality of electrolysis module 110 rotationally.

As such, the reverse voltage is not applied to electrodes of all the electrolysis modules 110 but may be applied only to electrodes of some of the electrolysis modules 110, and the reverse voltage may be applied to the plurality of electrolysis modules 110 rotationally, thereby slowing down deterioration of the electrodes due to the application of the reverse voltage.

Furthermore, application of the reverse voltage only to electrodes of some electrolysis modules 110 may slow down deterioration of the electrodes as compared to application of the reverse voltage to all the electrodes.

Referring to FIG. 6, the controller 150 determine one of the plurality of electrolysis modules 110 as the first electrolysis module and control no voltage to be applied across the electrodes of the first electrolysis module.

For example, when there are four electrolysis modules 110: electrolysis module A 111, electrolysis module B 112, electrolysis module C 113 and electrolysis module D 114, no voltage may be applied across the electrodes of one of the four electrolysis modules 110 in each stage.

In stage 1, when no voltage is applied to the electrolysis module A 111, the electrolysis module A 11 1may pass the water brought in and the remaining electrolysis modules 110 receive the forward voltage to generate sterilized water. Specifically, in stage 1, the electrolysis module A 111 may be the first electrolysis module, and the electrolysis modules B, C and D 112, 113 and 114 may be the second electrolysis modules.

After determining the first and second electrolysis modules in stage 1, the procedure may proceed to stage 2 after the lapse of a certain time.

In stage 2, the controller 150 controls no voltage to be applied to the electrolysis module B 112 and the forward voltage to be applied to the remaining electrolysis modules 110 to generate the sterilized water. Specifically, in stage 2, the electrolysis module B 112 may be the first electrolysis module, and the electrolysis modules A, C and D 111, 113 and 114 may be the second electrolysis modules.

The procedure may proceed to stage 3 after the lapse of the certain time. After the controller 150 determines the first and second electrolysis modules in stage 4, the procedure may proceed back to stage 1 after the lapse of the certain time.

Meanwhile, after no voltage is applied to the electrolysis module A 111, it is not always needed to apply no voltage to the electrolysis module B 112 in the next stage but there may be any different sequence of applying no voltage to the plurality of electrolysis module 110 rotationally.

As such, to generate the sterilized water, not all but some of the electrolysis modules 110 perform electrolysis, and as the plurality of electrolysis modules 110 take turns not to receive the voltage, a rate of scales being formed on the surfaces of the electrodes may slow down.

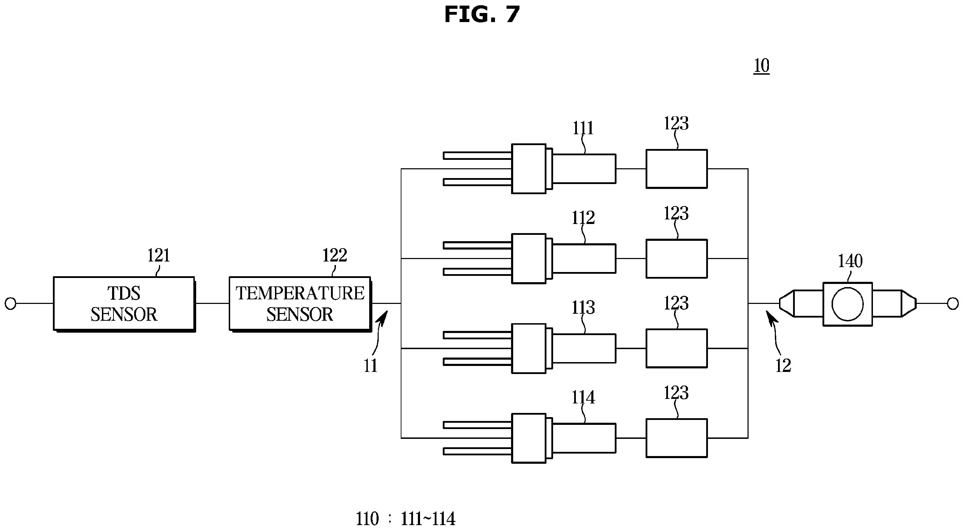

FIG. 7 illustrates positions of respective sensors in a water purifier, according to an embodiment of the disclosure.

Referring to FIG. 7, the TDS sensor 121 and the temperature sensor 122 may be arranged in the water inlet pipe 11.

The TDS sensor 121 may measure an amount of TDS contained in the water brought into the water inlet pipe 11. The TDS sensor 121 may send information about the measured amount of TDS to the controller 150.

The temperature sensor 122 may measure a water temperature of the water brought in through the water inlet pipe 11. The temperature sensor 122 may send information about the measured water temperature to the controller 150.

In an embodiment of the disclosure, the flow volume sensor 140 may be arranged in the water outlet pipe 12 to detect flow volume in the water outlet pipe 12. When a fluid flows in the water outlet pipe 12 as in a case that the water outlet valve of the water outlet pipe 12 is opened to discharge the sterilized water through the water outlet nozzle in the water outlet pipe 12, the flow volume sensor 140 may send information about the corresponding flow volume to the controller 150.

When the total flow volume detected after the last electrolysis performed by the electrolysis module 110 is equal to or larger than a threshold flow volume, the controller 150 may control the first electrolysis module of the plurality of electrolysis modules 110 not to electrolyze the water brought in, and control the second electrolysis module of the plurality of electrolysis modules 110 to electrolyze the water brought in.

In other words, the sterilized water generator 10 does not always generate the sterilized water but may start generating the sterilized water using some of the electrolysis modules 110 when the flow volume of water output from the sterilized water generator 10 reaches a certain amount after the sterilized water is generated using some of the electrolysis modules 110.

The threshold flow volume may be 200 L, but it is not limited thereto and the user may set the threshold flow volume in advance.

Furthermore, the sterilized water generator 10 may determine when to generate the sterilized water not based on the flow volume but based on a time. For example, when a threshold period of time elapses after the electrolysis module 110 performs the last electrolysis, the controller 150 may control the first electrolysis module of the plurality of electrolysis modules 110 not to electrolyze the water brought in, and control the second electrolysis module of the plurality of electrolysis modules 110 to electrolyze the water brought in.

The threshold period may be 30 days, but it is not limited thereto and the user may set the threshold period in advance.

The sterilized water generator 10 may include a plurality of current sensors 123. The plurality of current sensors 123 may be equipped in the plurality of electrolysis modules 110, respectively. Each of the plurality of current sensors 123 may measure a current flowing in the water passing each of the plurality of electrolysis modules 110.

The current value measured in each electrolysis module 110 may be different. For example, a value of the current flowing in the electrolysis module A 111 may differ from a value of the current flowing in the electrolysis module B 112.

The current sensor 123 may send information about the measured current to the controller 150.

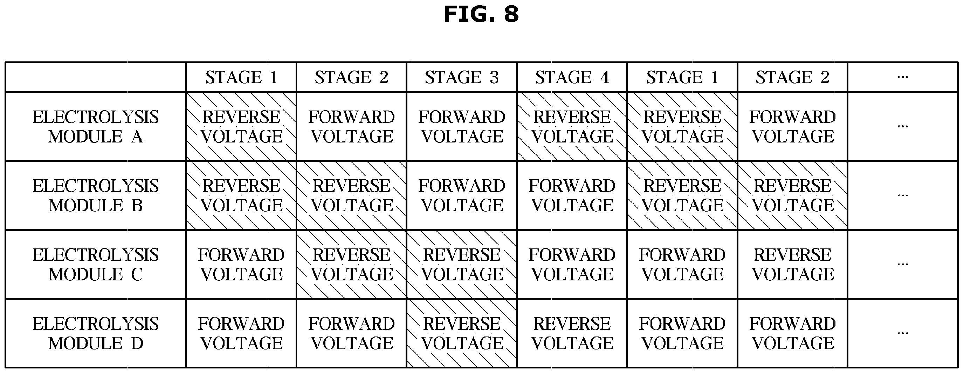

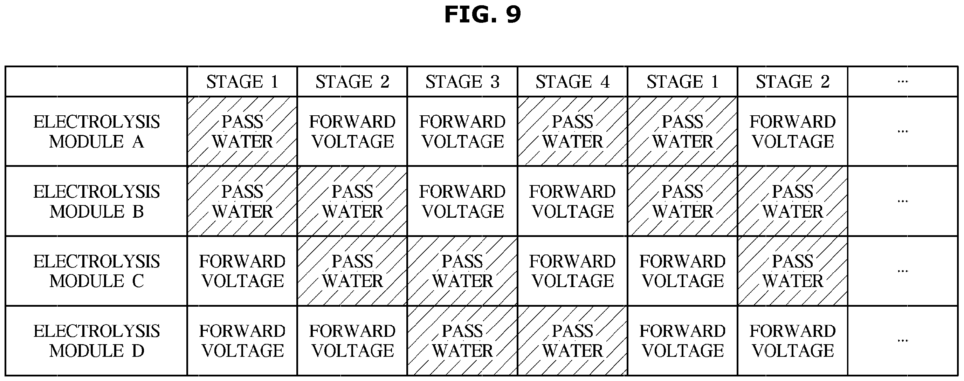

FIG. 8 is a table representing an example of applying a reverse voltage to two of the plurality of electrolysis modules 110 rotationally, according to an embodiment of the disclosure. FIG. 9 is a table representing an example of not applying a voltage to electrodes of two of the plurality of electrolysis modules 110 rotationally, according to an embodiment of the disclosure.

Referring to FIGS. 8 and 9, the controller 150 may control two of the plurality of the electrolysis modules 110 rotationally not to electrolyze the water brought in.

Specifically, the controller 150 may not control only one of the electrolysis modules 110 not to electrolyze the water brought in, but may control two or more electrolysis modules 110 not to perform electrolysis based on a condition of the tap water or a condition under which the sterilized water generator 10 is installed.

The controller 150 may determine the number of the first electrolysis modules based on the condition of tap water measured by the sensor. In other words, the controller 150 may determine the number of electrolysis modules 110 that do not electrolyze the water brought in among the plurality of electrolysis modules 110 based on information about the condition of the tap water.

When the tap water is in a good condition for electrolysis, the controller 150 may determine a relatively large number of electrolysis modules 110 among the plurality of electrolysis modules 110 as the first electrolysis modules and a relatively small number of electrolysis modules 110 as the second electrolysis modules.

On the other hand, when the tap water is in a poor condition for electrolysis, the controller 150 may determine a relatively small number of electrolysis modules 110 among the plurality of electrolysis modules 110 as the first electrolysis modules and a relatively large number of electrolysis modules 110 as the second electrolysis modules.

In this way, determining the number of first electrolysis modules that do not perform electrolysis based on the condition of the tap water may prevent too much water electrolysis or too lethargic water electrolysis, thereby maintaining a certain level of electrolysis performance.

The controller 150 may control the determined number of first electrolysis modules not to electrolyze the water brought in.

The more the amount of TDS in the tap water is, the better the electrolysis is performed. The controller 150 may determine the number of the first electrolysis modules based on the amount of TDSs in the tap water. For example, the controller 150 may determine a larger number of the first electrolysis modules the more the amount of TDSs is in the tap water.

The higher the water temperature of the tap water is, the better the electrolysis is performed. The controller 150 may determine the number of the first electrolysis modules based on the water temperature of the tap water. The controller 150 may determine a larger number of the first electrolysis modules the higher the water temperature of the tap water is.

The higher the magnitude of a current flowing in the water passing the electrolysis module 110 is, the better the electrolysis is performed. The controller 150 may determine the number of the first electrolysis modules based on the magnitude of a current flowing in the water passing the electrolysis module 110. That is, the controller 150 may determine a larger number of the first electrolysis modules the higher the value of the current flowing in the water passing the electrolysis module 110 is.

The tap water may be in a good condition or poor condition for electrolysis depending on an environment in which the sterilized water generator 10 is installed.

The controller 150 may determine the number of the first electrolysis modules based on outside environment information received by the communication module 130 from the server 200.

The communication module 130 may receive, from the server 200, season information of a region where the sterilized water generator 10 is located, and the controller 150 may determine the number of the first electrolysis modules based on the season information.

For example, when it is the summer in the region where the sterilized water generator 10 is installed, the water temperature is high, the condition of which is good for electrolysis, so the controller 150 may determine a relatively large number of the first electrolysis modules as compared to an occasion when the region where the sterilized water generator 10 is installed is in the winter.

The communication module 130 may receive, from the server 200, weather information of the region where the sterilized water generator 100 is located, and the controller 150 may determine the number of the first electrolysis modules based on the weather information.

For example, when the temperature in the region where the sterilized water generator 10 is installed is high, the controller 150 may determine a relatively large number of the first electrolysis modules as compared to an occasion when the temperature in the region where the sterilized water generator 10 is installed is low.

The communication module 130 may receive, from the server 200, water quality information of the region where the sterilized water generator 10 is located, and the controller 150 may determine the number of the first electrolysis modules based on the water quality information.

For example, when the tap water supplied to every household in the region where the sterilized water generator 10 is installed is hard water, the controller 150 may determine a larger number of the first electrolysis modules than when the tap water in the region where the sterilized water generator 10 is installed is soft water.

Referring to FIGS. 8 and 9, the controller 150 may determine two of the plurality of electrolysis modules 110 as the first electrolysis modules and control the first electrolysis modules rotationally not to electrolyze the water brought in. In this case, the controller 150 may rotationally change two of the plurality of electrolysis modules 110 to be the first electrolysis modules.

It may also be possible that the controller 150 may determine three of the plurality of electrolysis modules 110 as the first electrolysis modules based on tap water condition and surrounding environment information.

Referring to FIG. 8, when there are four electrolysis modules 110: electrolysis module A 111, electrolysis module B 112, electrolysis module C 113 and electrolysis module D 114, the reverse voltage may be applied to electrodes of two of the four electrolysis modules 110 in each stage.

In stage 1, when the reverse voltage is applied to the electrolysis module A 111 and the electrolysis module B 112, the forward voltage may be applied to the remaining electrolysis modules 110 to generate sterilized water. Specifically, in stage 1, the electrolysis module A 111 and the electrolysis module B 112 may be the first electrolysis modules, and the electrolysis modules C and D 113 and 114 may be the second electrolysis modules.

After determining the first and second electrolysis modules in stage 1, the procedure may proceed to stage 2 after the lapse of a certain time. In stage 2, the controller 150 may control the reverse voltage to be applied to the electrolysis module B 112 and the electrolysis module C 113 and control the forward voltage to be applied to the remaining electrolysis modules 110 to generate sterilized water. Specifically, in stage 2, the electrolysis module B 112 and the electrolysis module C 113 may be the first electrolysis modules, and the electrolysis modules A and D 111 and 114 may be the second electrolysis modules. The procedure may proceed to stage 3 after the lapse of the certain time.

After the controller 150 determines the first and second electrolysis modules in stage 4, the procedure may proceed back to stage 1 after the lapse of the certain time. In this way, the controller 150 may control two of the plurality of electrolysis modules 110 rotationally to receive the reverse voltage not to perform electrolysis on the water brought in while the sterilized water generator 100 is generating sterilized water.

Meanwhile, after the reverse voltage is applied to the electrolysis modules A and B 111 and 112, it is not always needed to apply the reverse voltage to the electrolysis modules B and C 112 and 113 in the next stage but there may be any different sequence and combination of applying the reverse voltage to the plurality of electrolysis module 110 rotationally.

Referring to FIG. 9, when there are four electrolysis modules 110: electrolysis module A 111, electrolysis module B 112, electrolysis module C 113 and electrolysis module D 114, no voltage may be applied to electrodes of two of the four electrolysis modules 110 in each stage.

In stage 1, when no voltage is applied to the electrolysis module A 111 and the electrolysis module B 112, the forward voltage may be applied to the remaining electrolysis modules 110 to generate sterilized water. Specifically, in stage 1, the electrolysis module A 111 and the electrolysis module B 112 may be the first electrolysis modules, and the electrolysis modules C and D 113 and 114 may be the second electrolysis modules.

After determining the first and second electrolysis modules in stage 1, the procedure may proceed to stage 2 after the lapse of a certain time. In stage 2, the controller 150 may control no voltage to be applied to the electrolysis module B 112 and the electrolysis module C 113 and control the forward voltage to be applied to the remaining electrolysis modules 110 to generate sterilized water. Specifically, in stage 2, the electrolysis module B 112 and the electrolysis module C 113 may be the first electrolysis modules, and the electrolysis modules A and D 111 and 114 may be the second electrolysis modules. The procedure may proceed to stage 3 after the lapse of the certain time.

After the controller 150 determines the first and second electrolysis modules in stage 4, the procedure may proceed back to stage 1 after the lapse of the certain time. In this way, the controller 150 may control two of the plurality of electrolysis modules 110 rotationally to receive no voltage to prevent electrolysis from being performed on the water brought in while the sterilized water generator 100 is generating sterilized water.

Meanwhile, after no voltage is applied to the electrolysis modules A and B 111 and 112, it is not always needed to apply no voltage to the electrolysis modules B and C 112 and 113 in the next stage but there may be any different sequence and combination of applying no voltage to the plurality of electrolysis module 110 rotationally.

It may also be possible that the controller 150 may determine all of the plurality of electrolysis modules 110 as the second electrolysis modules based on tap water condition and surrounding environment information. In this case, all the electrolysis modules 110 may perform electrolysis on the water brought in.

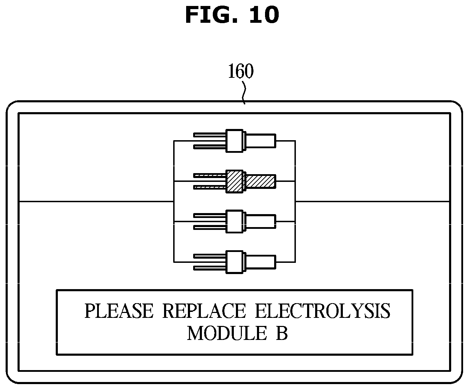

FIG. 10 illustrates a display displaying an indication of one of the plurality of electrolysis modules 110 required to be replaced, according to an embodiment of the disclosure.

Referring to FIG. 10, the controller 150 may control the display 160 to display an indication of the electrolysis module 110 that needs to be replaced.

For example, the controller 150 may control the display 160 to display an expression "please replace electrolysis module B" when the electrolysis module B 112 needs to be replaced.

Furthermore, the controller 150 may control the display 160 to display a position of the electrolysis module B 112 that needs to be replaced.

The user may then understand which of the plurality of electrolysis modules 110 needs to be replaced and where the electrolysis module 110 that needs to be replaced is relatively positioned by checking the screen of the display 160.

The information display method according to the aforementioned and following embodiments of the disclosure may be implemented in the form of a program operated by the sterilized water generator 10.

The program as herein used may include program instructions, data files, data structures, etc., separately or in combination. The program may be designed and produced with machine language codes or high-level language codes. The program may be specially designed to implement the method, or may be implemented by using various available functions or definitions known to those of ordinary skill in the art in the computer software field.

The memory 180 may store programs for performing the aforementioned and following operations, and a processor included in the controller 150 may execute the program. In a case that the memory 180 and the processor are each provided in the plural, they may be integrated in a single chip or physically distributed. The memory 180 may include a volatile memory for temporarily storing data, such as a static random access memory (SRAM), a dynamic random access memory (DRAM), or the like. The memory 180 may also include a non-volatile memory for storing a control program and control data for a long time, such as a read-only memory (ROM), an erasable programmable ROM (EPROM), an electrically erasable programmable ROM (EEPROM), etc. The processor may include many different logic circuits and operation circuits, process data according to the program provided in the memory 180, and generate control signals according to the processing results.

There may be more or fewer components to correspond to the functions of the aforementioned components. Furthermore, it will be obvious to those of ordinary skill in the art that the relative positions of the components may be changed to correspond to the system performance or structure.

A method of controlling the sterilized water generator 10 will now be described in accordance with an embodiment of the disclosure. As for the method, the sterilized water generator 10 in the aforementioned embodiments of the disclosure may be used. Hence, what are described above with reference to FIGS. 1 to 10 may be equally applied in the following method of controlling the sterilized water generator 10.

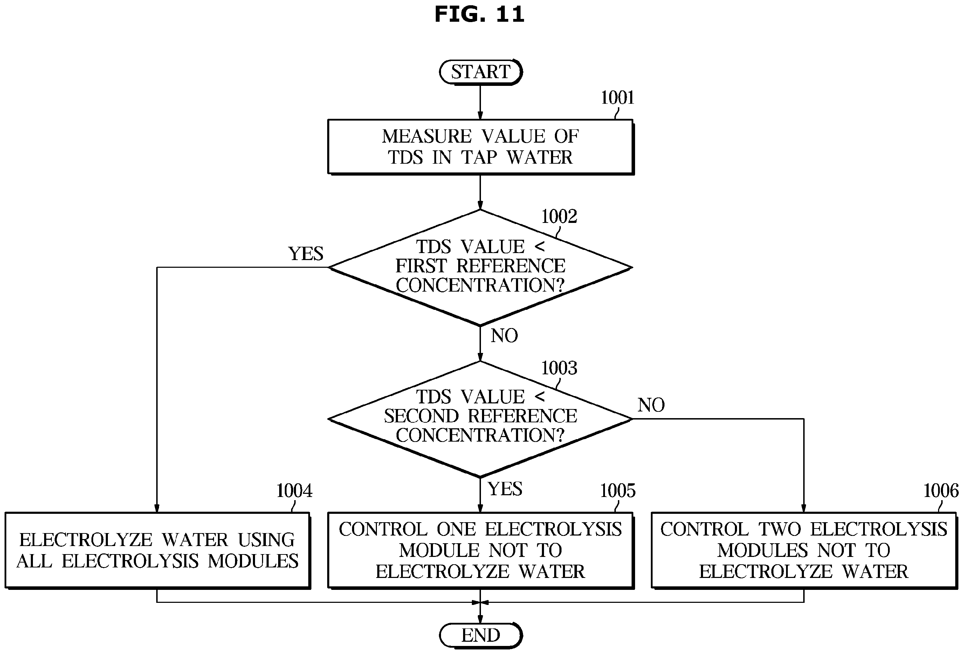

FIG. 11 is a flowchart illustrating a procedure for determining the number of first electrolysis modules based on a TDS value of the tap water, according to an embodiment of the disclosure.

Referring to FIG. 11, the TDS sensor 121 may measure a TDS value of the tap water brought into the water inlet pipe 11. The TDS sensor 121 may send information about the measured TDS value to the controller 150.

The controller 150 may determine whether the TDS value is smaller than a first reference concentration, in 1002.

The first reference concentration may be an amount of TDSs that becomes a basis to control all the plurality of electrolysis modules 110 to electrolyze the water brought in. Specifically, the first reference concentration may be 80 ppm, without being limited thereto.

When the TDS value is smaller than the first reference concentration (yes in 1002), the controller 150 may determine all the electrolysis modules 110 as the second electrolysis modules and use the all electrolysis modules 110 to electrolyze water, in 1004. In other words, the controller 150 may not determine any of the electrolysis modules 110 as the first electrolysis module while determining all the electrolysis modules 110 as the second electrolysis modules. This is to maintain a certain level of sterilized water generation performance of the sterilized water generator 10 by performing electrolysis using all the electrolysis modules 110 in a poor tap water condition for water electrolysis due to a low TDS value of the tap water.

When the TDS value is equal to or greater than the first reference concentration (no of 1002), the controller 150 may determine whether the TDS value is smaller than a second reference concentration, in 1003.

The second reference concentration may be an amount of TDSs that becomes a basis to control one of the plurality of electrolysis modules 110 not to electrolyze the water brought in. Specifically, the second reference concentration may be 160 ppm, without being limited thereto.

When the TDS value is smaller than the second reference concentration (yes in 1003), the controller 150 may determine one of the electrolysis modules 110 as the first electrolysis module and use the remaining electrolysis modules 110 to electrolyze the water, in 1005.

In this case, the controller 150 may apply the reverse voltage to one of the plurality of electrolysis modules 110 rotationally or may not apply any voltage to electrodes of one of the plurality of electrolysis modules 110 rotationally.

When the TDS value is equal to or greater than the second reference concentration (no in 1003), the controller 150 may determine two of the electrolysis modules 110 as the first electrolysis module and use the remaining electrolysis modules 110 to electrolyze the water, in 1006.

In this case, the controller 150 may apply the reverse voltage to two of the plurality of electrolysis modules 110 rotationally or may not apply any voltage to electrodes of two of the plurality of electrolysis modules 110 rotationally.

As such, the sterilized water generator 10 in an embodiment of the disclosure may determine the number of electrolysis modules 110 to perform electrolysis on the water brought in based on the TDS value of the tap water, thereby maintaining a certain level of electrolysis performance of the water purifier 100.

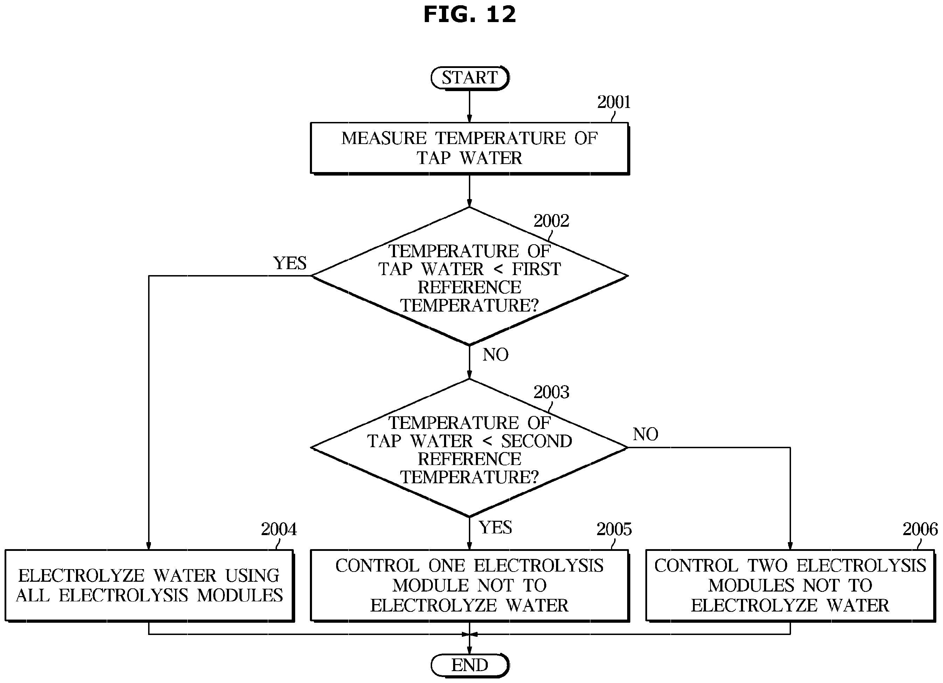

FIG. 12 is a flowchart illustrating a procedure for determining the number of first electrolysis modules based on water temperature of tap water, according to an embodiment of the disclosure;

Referring to FIG. 12, the temperature sensor 122 may measure temperature of the tap water brought into the water inlet pipe 11, in 2001. The temperature sensor 122 may send information about the measured temperature of the tap water to the controller 150.

The controller 150 may determine whether the temperature of the tap water is lower than a first reference temperature, in 2002.

The first reference temperature may be a temperature of the tap water that becomes a basis to control all the plurality of electrolysis modules 110 to electrolyze the water brought in. Specifically, the first reference temperature may be 10 °C, without being limited thereto.

When the temperature of the tap water is lower than the first reference temperature (yes in 2002), the controller 150 may determine all the electrolysis modules 110 as the second electrolysis modules and use the all electrolysis modules 110 to electrolyze water, in 2004. In other words, the controller 150 may not determine any of the electrolysis modules 110 as the first electrolysis module while determining all the electrolysis modules 110 as the second electrolysis modules. This is to maintain a certain level of sterilized water generation performance of the sterilized water generator 10 by performing electrolysis using all the electrolysis modules 110 in a poor tap water condition for water electrolysis due to low temperature of the tap water.

When the temperature of the tap water is equal to or higher than the first reference temperature (no of 2002), the controller 150 may determine whether the temperature of the tap water is lower than a second reference temperature, in 2003.

The second reference temperature may be a temperature of the tap water that becomes a basis to control one of the plurality of electrolysis modules 110 not to electrolyze the water brought in. Specifically, the second reference temperature may be 20 °C, without being limited thereto.

When the temperature of the tap water is lower than the second reference temperature (yes in 2003), the controller 150 may determine one of the electrolysis modules 110 as the first electrolysis module and use the remaining electrolysis modules 110 to electrolyze water, in 2005.

In this case, the controller 150 may apply the reverse voltage to one of the plurality of electrolysis modules 110 rotationally or may not apply any voltage to electrodes of one of the plurality of electrolysis modules 110 rotationally.

When the temperature of the tap water is equal to or higher than the second reference temperature (no in 2003), the controller 150 may determine two of the electrolysis modules 110 as the first electrolysis modules and use the remaining electrolysis modules 110 to electrolyze water, in 2006.

In this case, the controller 150 may apply the reverse voltage to two of the plurality of electrolysis modules 110 rotationally or may not apply any voltage to electrodes of two of the plurality of electrolysis modules 110 rotationally.

As such, the sterilized water generator 10 in an embodiment of the disclosure may determine the number of electrolysis modules 110 to perform electrolysis on the water brought in based on the temperature of the tap water, thereby maintaining a certain level of electrolysis performance of the water purifier 100.

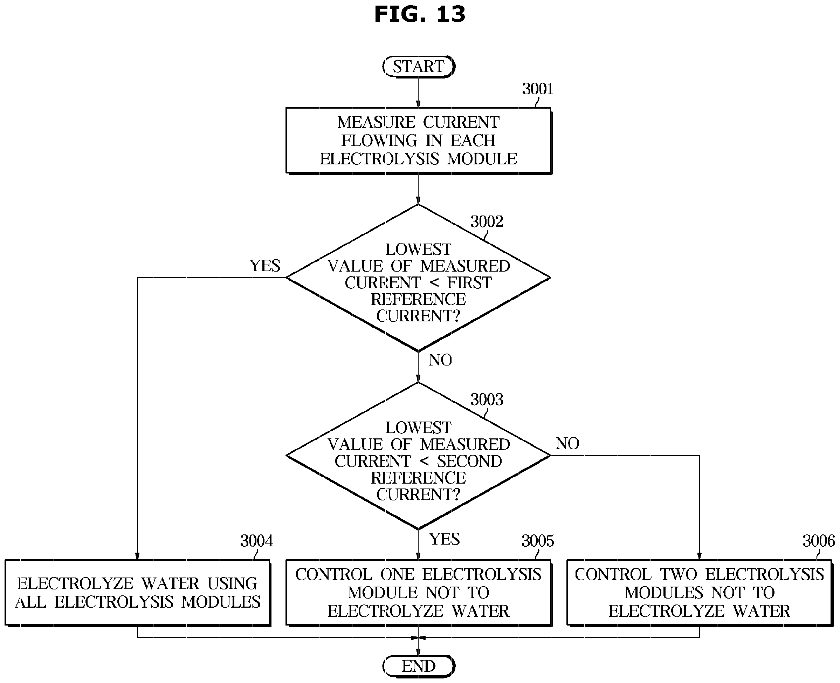

FIG. 13 is a flowchart illustrating a procedure for determining the number of first electrolysis modules based on a current flowing in each of the electrolysis modules 100, according to an embodiment of the disclosure.

Referring to FIG. 13, the sterilized water generator 10 may determine the number of electrolysis modules 110 that do not perform electrolysis based on the lowest of values of currents flowing in the respective electrolysis modules 110.

A current value that becomes a basis to determine the number of the first electrolysis modules is not, however, limited to the lowest value of the currents flowing in the respective electrolysis modules 110. For example, a current value that becomes a basis to determine the number of the first electrolysis modules may be the highest value or an average value of currents flowing in the respective electrolysis modules.

Each of the plurality of current sensors 123 may measure a value of a current flowing in the water passing each of the plurality of electrolysis modules 110, in 3001. The current sensor 123 may send information about the measured current to the controller 150.

The controller 150 may determine whether the lowest value of the measured currents is less than a first reference current, in 3002.

The first reference current may be a current value that becomes a basis to control all the plurality of electrolysis modules 110 to electrolyze the water brought in. Specifically, the first reference current may be 0.45 A, without being limited thereto.

When the lowest value of the measured currents is smaller than the first reference current (yes in 3002), the controller 150 may determine all the electrolysis modules 110 as the second electrolysis modules and use the all electrolysis modules 110 to electrolyze water, in 3004. In other words, the controller 150 may not determine any of the electrolysis modules 110 as the first electrolysis module while determining all the electrolysis modules 110 as the second electrolysis modules. This is to maintain a certain level of sterilized water generation performance of the sterilized water generator 10 by performing electrolysis using all the electrolysis modules 110 in a poor tap water condition for water electrolysis due to a low value of current flowing in the tap water.

When the lowest value of the measured currents is equal to or larger than the first reference current (no of 3002), the controller 150 may determine whether the lowest value of the measured currents is smaller than a second reference current, in 3003.

The second reference current may be a current value that becomes a basis to control one of the plurality of electrolysis modules 110 not to electrolyze the water brought in. Specifically, the second reference current may be 0.9 A, without being limited thereto.

When the lowest value of the measured currents is smaller than the second reference current (yes in 3003), the controller 150 may determine one of the electrolysis modules 110 as the first electrolysis module and use the remaining electrolysis modules 110 to electrolyze water, in 3005.

In this case, the controller 150 may apply the reverse voltage to one of the plurality of electrolysis modules 110 rotationally or may not apply any voltage to electrodes of one of the plurality of electrolysis modules 110 rotationally.

When the lowest value of the measured currents is equal to or greater than the second reference current (no in 3003), the controller 150 may determine two of the electrolysis modules 110 as the first electrolysis module and use the remaining electrolysis modules 110 to electrolyze water, in 3006.

In this case, the controller 150 may apply the reverse voltage to two of the plurality of electrolysis modules 110 rotationally or may not apply any voltage to electrodes of two of the plurality of electrolysis modules 110 rotationally.

As such, the sterilized water generator 10 in an embodiment of the disclosure may determine the number of electrolysis modules 110 to perform electrolysis on the water brought in based on the lowest current value of the values of the currents flowing in the plurality of electrolysis modules 110, thereby maintaining a certain level of electrolysis performance of the sterilized water generator 10.

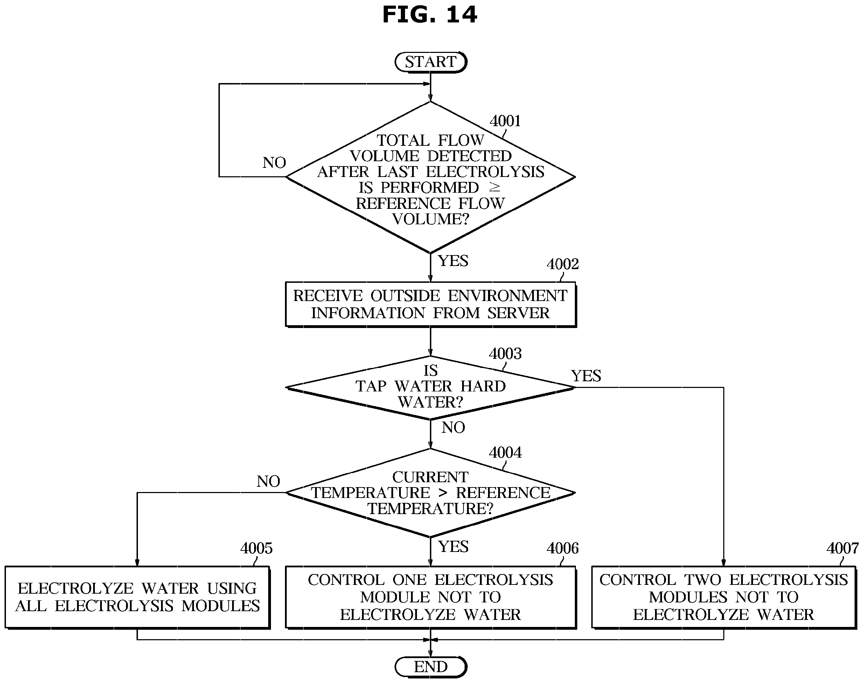

FIG. 14 is a flowchart illustrating a procedure for determining the number of first electrolysis modules based on outside environment information received from a server, according to an embodiment of the disclosure.

Referring to FIG. 14, the controller 150 may determine whether total flow volume detected after the last electrolysis is performed is equal to or larger than a reference flow volume, in 4001.

When the total flow volume detected after the last electrolysis is performed is smaller than the reference flow volume (no of 4001), the sterilized water generator 10 may not perform electrolysis on the water brought in. That is, the sterilized water generator 10 may not generate sterilized water.

When the total flow volume detected after the last electrolysis is performed is equal to or larger than the reference flow volume (yes of 4001), the controller 150 may control the communication module 130 to receive outside environment information from the server 200, in 4002. The received outside environment information may include water quality information of a region where the sterilized water generator 10 is installed, weather information of the region where the sterilized water generator 10 is installed, etc.

The controller 150 may determine whether the tap water is hard water based on the outside environment information received from the server 200, in 4003. Whether the tap water is hard water or soft water may vary depending on a standard of a region where the sterilized water generator 10 is installed even with the same ingredients of the tap water.

When the tap water is hard water (yes in 4003), the controller 150 may determine two of the electrolysis modules 110 as the first electrolysis modules and use the remaining electrolysis modules 110 to electrolyze water, in 4007.

In this case, the controller 150 may apply the reverse voltage to two of the plurality of electrolysis modules 110 rotationally or may not apply any voltage to electrodes of two of the plurality of electrolysis modules 110 rotationally.

When the tap water is not hard water (no of 4003), i.e., the tap water is soft water, the controller 150 may determine whether the current temperature of the region where the sterilized water generator 10 is installed exceeds a reference temperature based on the outside environment information received from the server 200, in 4004.

The reference temperature may be a temperature value that becomes a basis to control all of the plurality of electrolysis modules 110 to electrolyze the water brought in.

When the current temperature of the region where the sterilized water generator 10 is installed is lower than the reference temperature (no in 4004), the controller 150 may determine all the electrolysis modules 110 as the second electrolysis modules and use the all electrolysis modules 110 to electrolyze water, in 4005. In other words, the controller 150 may not determine any of the electrolysis modules 110 as the first electrolysis module while determining all the electrolysis modules 110 as the second electrolysis modules.

When the current temperature of the region where the sterilized water generator 10 is installed exceeds the reference temperature (yes of 4004), the controller 150 may determine one of the electrolysis modules 110 as the first electrolysis module and use the remaining electrolysis modules 110 to electrolyze water, in 4006.

In this case, the controller 150 may apply the reverse voltage to one of the plurality of electrolysis modules 110 rotationally or may not apply any voltage to electrodes of one of the plurality of electrolysis modules 110 rotationally.

As such, the sterilized water generator 10 in an embodiment of the disclosure may determine the number of electrolysis modules 110 to perform electrolysis on the water brought in based on the environment of the region where the sterilized water generator 10 is installed, thereby maintaining a certain level of electrolysis performance of the sterilized water generator 10.

The embodiments of the disclosure may be implemented in the form of a recording medium for storing instructions to be carried out by a computer. The instructions may be stored in the form of program codes, and when executed by a processor, may generate program modules to perform operation in the embodiments of the disclosure. The recording media may correspond to computer-readable recording media.

The computer-readable recording medium includes any type of recording medium having data stored thereon that may be thereafter read by a computer. For example, it may be a ROM, a RAM, a magnetic tape, a magnetic disk, a flash memory, an optical data storage device, etc. The recording medium may be the memory 180 of the water purifier 100.

Several embodiments of the disclosure have been described above, but a person of ordinary skill in the art will understand and appreciate that various modifications can be made without departing from the scope of the disclosure. Thus, it will be apparent to those or ordinary skill in the art that the true scope of technical protection is only defined by the following claims.

## Claims

1. A sterilized water generator comprising:
a water inlet pipe through which water flows to the sterilized water generator;
a water outlet pipe through which sterilized water flows from the sterilized water generator;
a plurality of electrolysis modules arranged in parallel to each other, along a flow path, between the water inlet pipe and the water outlet pipe, the plurality of electrolysis modules configured to electrolyze the water flowing through the water inlet pipe to produce the sterilized water; and
a controller configured to
control a forward voltage not to be applied to a first electrolysis module of the plurality of electrolysis modules,
control the forward voltage to be applied to a second electrolysis module of the plurality of electrolysis modules, and
change electrolysis modules corresponding to the first and second electrolysis modules of the plurality of electrolysis modules based on a predetermined lapse of time.

2. The sterilized water generator of claim 1, wherein the controller is configured to control a reverse voltage so that the reverse voltage is applied to an electrode of the first electrolysis module.

3. The sterilized water generator of claim 1, wherein the controller is configured to control a voltage so that the voltage is not applied to an electrode of the first electrolysis module.

4. The sterilized water generator of claim 1, wherein the controller is configured to control the forward voltage not to be applied to the first electrolysis module rotationally.

5. The sterilized water generator of claim 1, further comprising:
a sensor module configured to measure a condition of the water flowing through the water inlet pipe,
wherein the controller is configured to determine a number of first electrolysis modules based on the condition of the water flowing through the water inlet pipe, and control the forward voltage not to be applied to the determined number of first electrolysis modules.

6. The sterilized water generator of claim 5, wherein the sensor module is configured to measure an amount of total dissolved solids (TDSs) in the water flowing through the water inlet pipe, and
wherein the controller is configured to determine a number of the first electrolysis modules based on the amount of TDSs in the water flowing through the water inlet pipe.

7. The sterilized water generator of claim 5, wherein the sensor module is configured to measure water temperature of the water flowing through the water inlet pipe, and
wherein the controller is configured to determine a number of the first electrolysis modules based on the water temperature of the water flowing through the water inlet pipe.

8. The sterilized water generator of claim 5, wherein the sensor module is configured to measure the magnitude of a current flowing in the water passing the plurality of electrolysis modules, and
wherein the controller is configured to determine a number of the first electrolysis modules based on the magnitude of the current.

9. The sterilized water generator of claim 1, further comprising: a communication module configured to receive outside environment information from a server,
wherein the controller is configured to determine a number of the first electrolysis modules based on the outside environment information, and control the forward voltage not to be applied to the determined number of first electrolysis modules.

10. The sterilized water generator of claim 9, wherein the communication module is configured to receive, from the server, weather information of a region in which the sterilized water generator is located, and
wherein the controller is configured to determine a number of the first electrolysis modules based on the weather information.

11. The sterilized water generator of claim 9, wherein the communication module is configured to receive, from the server, water quality information of a region in which the sterilized water generator is located, and
wherein the controller is configured to determine a number of the first electrolysis modules based on the water quality information.

12. The sterilized water generator of claim 1, further comprising: a flow volume sensor configured to detect flow volume of the water brought in,
wherein the controller is configured to , when flow volume of the sterilized water flowing out after the water is electrolyzed is equal to or larger than a reference flow volume, control the forward voltage not to be applied to the first electrolysis module of the plurality of electrolysis modules and control the forward voltage to be applied to the second electrolysis module of the plurality of electrolysis modules.

13. The sterilized water generator of claim 1, further comprising: a display,
wherein the controller is configured to control the display to display an indication of an electrolysis module required to be replaced among the plurality of electrolysis modules.

14. The sterilized water generator of claim 1, further comprising:
a notifier, wherein the controller is configured to control the notifier to output an alarm indicating an electrolysis module causing a malfunction or fault among the plurality of electrolysis modules.

15. A water purifier comprising:
a first connection flow path through which water flows to the water purifier;
a second connection flow path through which water flows from the water purifier;
a filter arranged along the first connection flow path to filter the water; and
a sterilized water generator configured to generate sterilized water,
wherein the sterilized water generator comprises
a water inlet pipe connected to the first connection flow path;
a water outlet pipe connected to the second connection flow path;
a plurality of electrolysis modules arranged parallel to each other, between the water inlet pipe and the water outlet pipe and the plurality of electrolysis modules configured to electrolyze the water flowing through the water inlet pipe to generate the sterilized water; and
a controller configured to
control a forward voltage not to be applied to a first electrolysis module of the plurality of electrolysis modules,
control the forward voltage to be applied to a second electrolysis module of the plurality of electrolysis modules, and
change electrolysis modules corresponding to the first and second electrolysis modules of the plurality of electrolysis modules based on a predetermined lapse of time.
